# EUROPÄISCHE PATENTSCHRIFT

(11) **EP 0 497 210 B1**
(45) Veröffentlichungstag und Bekanntmachung des Hinweises auf die Patenterteilung: **14.06.1995**
(21) Anmeldenummer: 92101077.3
(22) Anmeldetag: 23.01.1992
(51) Int. Cl.: C07C 215/10, C07C 213/02

(54) **Verfahren zur Herstellung von Dialkylaminopropandiol**
Process for the preparation of dialkylaminopropane diol
Procédé pour la préparation de dialkylaminopropane diol

(30) Priorität: 28.01.1991 DE 4102394
(43) Veröffentlichungstag der Anmeldung: 05.08.1992
(73) Patentinhaber: HOECHST AKTIENGESELLSCHAFT, 65926 Frankfurt am Main (DE)
(72) Erfinder: Aigner, Rudolf, W-8269 Burgkirchen (DE); Müller, Günter, Dr., W-8269 Burgkirchen (DE); Scholz, Hans Jürgen, Dr., W-8755 Alzenau (DE); Wehle, Detlef, Dr., W-6272 Niedernhausen (DE)

(56) Entgegenhaltungen:
- EP-A- 0 025 083
- DE-B- 1 004 194
- US-A- 2 042 621
- US-A- 2 147 226

## Beschreibung

Die Erfindung betrifft ein Verfahren zur Herstellung von Dialkylaminopropandiol durch Umsetzung von Monohalogenpropandiol mit überschüssigem Dialkylamin in Gegenwart von Wasser.

Ein solches Verfahren ist in der US-Patentschrift 2,147,226 beschrieben, wobei Monochlorpropandiol (Glycerin-monochlorhydrin) und Dimethylamin als Ausgangsverbindungen eingesetzt werden. Die nachstehende Gleichung unter Verwendung der genannten Ausgangsverbindungen soll die Reaktion veranschaulichen: 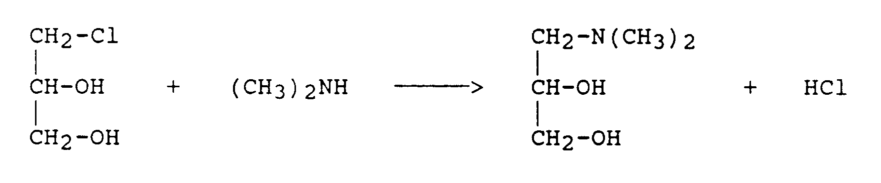
Gemäß US-Patentschrift 2,147,226 wird das Dimethylamin in einer Menge von bis zu 1,5 mol pro mol Glycerin-monochlorhydrin eingesetzt. Die exotherm verlaufende Umsetzung wird bei einer Temperatur von 20 bis 50 °C und in Gegenwart von relativ viel Wasser durchgeführt (im Ausführungsbeispiel wird das Dimethylamin in Form einer 24,5%igen wäßrigen Lösung eingesetzt, das ist 75,5 Gew.-% Wasser, bezogen auf die Mischung aus Dimethylamin und Wasser). Die Bindung der bei der Umsetzung freiwerdenden Chlorwasserstoffsäure erfolgt mit einem Alkalimetallhydroxid, das ebenfalls in Form einer wäßrigen Lösung eingesetzt wird. Bei diesem Verfahren wird also mit einer niedrigen Konzentration an Dimethylamin und infolgedessen mit einer großen Wassermenge gearbeitet. Dies stellt einen wesentlichen Mangel dieses Verfahrens dar. Ein weiterer Nachteil liegt darin, daß die Reaktionszeit relativ lang ist und die Ausbeute an Dimethylaminopropandiol, bezogen auf Glycerin-monochlorhydrin, trotzdem niedrig ist. Aus dem einzigen Beispiel ergibt sich zwar eine Ausbeute von 98 % der Theorie. Dieser hohe Wert konnte aber durch Nacharbeiten des Beispiels nicht bestätigt werden.

In der genannten US-Patentschrift 2,147,226 wird eingangs ein Stand der Technik erwähnt, wonach Dimethylaminopropandiol im Laboratorium durch Erhitzen von Glycerin-monochlorhydrin und Dimethylamin in einem geschlossenen Rohr auf 100 °C hergestellt worden ist. Bei dieser relativ hohen Temperatur bilden sich unerwünschte Nebenprodukte. Im Vergleich zu dieser wasserfreien Herstellungsart ist also jene der genannten US-Patentschrift vorteilhafter.

Der Erfindung liegt demnach die Aufgabe zugrunde, das aus der US-Patentschrift 2,147,226 bekannte und auf niedriger Reaktionstemperatur basierende Verfahren, insbesondere bezüglich Wassermenge, Reaktionszeit und Ausbeute, zu verbessern.

Das erfindungsgemäße Verfahren ist dadurch gekennzeichnet, daß das Dialkylamin in einer Menge von mindestens 2,5 mol pro mol Monohalogenpropandiol, vorzugsweise in einer Menge von 3 bis 6 mol pro mol Monohalogenpropandiol, eingesetzt und die Umsetzung in Gegenwart von 25 bis 70 Gew.-% Wasser, vorzugsweise 30 bis 60 Gew.-% Wasser, bezogen auf die Mischung aus Dialkylamin und Wasser, und bei einer Temperatur von 20 bis 80 °C, vorzugsweise 30 bis 60 °C, durchgeführt wird und das angestrebte Dialkylaminopropandiol aus dem Umsetzungsprodukt gewonnen wird.

Beim erfindungsgemäßen Verfahren wird also die Umsetzung des Dialkylamins und des Halogenpropandiols in Gegenwart einer ganz bestimmten Wassermenge durchgeführt. So kommt die Reaktion bei der angegebenen Temperatur im Falle von weniger als 25 Gew.-% Wasser, bezogen auf das Gemisch aus Dialkylamin und Wasser, nach einer mehr oder weniger langen Reaktionszeit einfach zum Stillstand. Mit mehr als 70 Gew.-% Wasser wird zwar eine relativ kurze Reaktionszeit erreicht, aber die Raum-Zeit-Ausbeute verschlechtert. Die Wassermenge (das wäßrige Medium) stellt also eine kritische Größe des erfindungsgemäßen Verfahrens dar und liegt, wie oben angegeben, bei 25 bis 70 Gew.-%, vorzugsweise 30 bis 60 Gew.-%, Gewichtsprozente bezogen auf die Summe an eingesetztem Dialkylamin und Wasser.

Beim erfindungsgemäßen Verfahren wird ferner das Dialkylamin in einer Menge von mindestens 2,5 mol pro mol Halogenpropandiol eingesetzt. Bei einem niedrigeren Molverhältnis werden bereits unerwünschte Nebenprodukte gebildet. Die obere Dialkylaminmenge kann in einem weiten Bereich variieren. In der Regel wird man nicht mehr als 6 mol Dialkylamin pro mol Halogenpropandiol einsetzen. Die Dialkylaminmenge liegt demnach vorzugsweise bei 3 bis 6 mol Dialkylamin pro mol Monohalogenpropandiol.

Die auf die angegebene Wassermenge und Dialkylaminmenge abgestimmte Reaktionstemperatur zur Erreichung der angestrebten Effekte liegt bei 20 bis 80 °C. Unter 20 °C ist die Reaktionszeit bereits relativ lang und über 80 °C kann es zur Bildung von Nebenprodukten kommen. Die bevorzugte Reaktionstemperatur ist daher 30 bis 60 °C.

Was die Bindung der bei der Umsetzung sich bildenden Halogenwasserstoffsäure betrifft, so werden dazu die anwesenden Aminverbindungen herangezogen. Die Bindung der Halogenwasserstoffsäure erfolgt also beim erfindungsgemäßen Verfahren durch das im System selbst anwesende Amin und nicht durch Alkalimetallhydroxide (das heißt basische Fremdverbindungen), wie beim Stand der Technik.

Zur Durchführung des erfindungsgemäßen Verfahrens werden das Dialkylamin und das Halogenpropandiol im angegebenen Molverhältnis und bei der angegebenen Wassermenge und Reaktionstemperatur in Kontakt gebracht. Die Art des Inkontaktbringens ist an sich nicht kritisch. Die folgenden Durchführungsformen (1 bis 4) sind bevorzugt: (1) Das gesamte Dialkylamin, Halogenpropandiol und Wasser werden in ein Reaktionsgefäß gegeben und so lange bei der angegebenen Temperatur gehalten, bis alles Halogenpropandiol umgesetzt ist. (2) Das gesamte Dialkylamin und das Wasser werden vorgelegt. Die Mischung wird auf Reaktionstemperatur gebracht, worauf bei dieser Temperatur das Halogenpropandiol als solches (portionsweise oder kontinuierlich) dazugegeben wird. Nach der Zugabe wird die Mischung noch weiter bei Reaktionstemperatur zur Nachreaktion gehalten. (3) Das gesamte Halogenpropandiol und das Wasser werden vorgelegt. Die Mischung wird auf Reaktionstemperatur gebracht, worauf bei dieser Temperatur das Dialkylamin als solches dazugegeben wird. Nach der Zugabe wird die Mischung noch weiter bei Reaktionstemperatur zur Nachreaktion gehalten. (4) Das gesamte Halogenpropandiol wird vorgelegt und auf Reaktionstemperatur gebracht, worauf bei dieser Temperatur das Dialkylamin und das Wasser, getrennt voneinander oder gemeinsam in Form einer wäßrigen Dialkylaminlösung dazugegeben werden. Nach der Zugabe wird die Mischung noch weiter bei Reaktionstemperatur zur Nachreaktion gehalten.
Die Reaktion zwischen dem Dialkylamin und Halogenpropandiol ist exotherm. Im Falle einer niedrigsiedenden Dialkylaminverbindung, wie Dimethylamin mit einem Siedepunkt von 7 °C bei Normaldruck, wird man die Umsetzung in einem Druckgefäß durchführen, da bei den genannten Umsetzungstemperaturen mit mehr oder weniger hohen Dampfdrücken zu rechnen ist. Die Reaktionszeit liegt bei 2 bis 10 Stunden und hängt vor allem von der gewählten Wassermenge und Reaktionstemperatur ab.

Sofern eine Aufarbeitung des nach dem erfindungsgemäßen Verfahren erhaltenen Umsetzungsproduktes und die Gewinnung der Dialkylaminopropandiol-Verbindung gewünscht werden, wird dies bevorzugt nach der folgenden Methode durchgeführt: Vom Umsetzungsprodukt, das im wesentlichen aus Dialkylaminopropandiol · HX (X = Halogen), dem überschüssig eingesetzten Dialkylamin und Wasser besteht, werden zunächst das Dialkylamin und das Wasser destillativ abgetrennt, worauf das Produkt Dialkylaminopropandiolhydrohalogenid in hoher Reinheit vorliegt. Zur Freisetzung des Dialkylaminopropandiols wird die Hydrohalogenidverbindung zwecks Neutralisation mit etwa 1 Moläquivalent Alkalimetallhydroxid, vorzugsweise Kalium- oder Natriumhydroxid, zweckmäßigerweise in Form einer 30- bis 60gew.%igen wäßrigen Lösung versetzt, wobei die Temperatur der Mischung durch Kühlung auf vorzugsweise 20 bis 60 °C gehalten wird. Aus der nun vorliegenden Mischung aus Dialkylaminopropandiol, Wasser und Salz kann das Dialkylaminopropandiol (zweckmäßigerweise nach Abfiltrieren oder Abzentrifugieren des Hauptteils des Salzes) einfach durch destillative Aufarbeitung gewonnen werden. Da das Dialkylaminopropandiol in der genannten Mischung in hoher Reinheit vorliegt, ist die destillative Isolierung des Dialkylaminopropandiols oft gar nicht erforderlich. In diesem Falle kann auf die relativ aufwendigen Schritte, Filtrieren oder Zentrifugieren, gegebenenfalls Waschen, und schließlich Destillieren, verzichtet werden. Auch das oben erwähnte, in hoher Reinheit erhaltene Dialkylaminopropandiolhydrohalogenid stellt bereits ein vorteilhaftes Ausgangsprodukt für Folgereaktionen dar.

Die Ausgangsverbindungen der erfindungsgemäßen Umsetzung sind Monohalogenpropandiol und Dialkylamin. Unter Dialkylamin werden im Rahmen der vorliegenden Erfindung die niedrigen Dialkylamine verstanden, vorzugsweise Dimethylamin und Diethylamin. All diese Verbindungen sind bekannt und im Handel erhältlich. Die genannten Dialkylamine sind Flüssigkeiten mit einem Siedepunkt von 7 °C (Dimethylamin) und 50 °C (Diethylamin) bei Normaldruck. Das besonders bevorzugte Dialkylamin ist das Dimethylamin. Halogenpropandiol liegt bekanntlich in zwei Strukturformen vor, und zwar als 3-Halogenpropan-1,2-diol oder als 2-Halogenpropan-1,3-diol, wobei die erstgenannte Verbindung die viel häufigere ist. Die nachstehenden Formeln sollen die beiden Strukturen veranschaulichen, wobei X für ein Halogen steht: 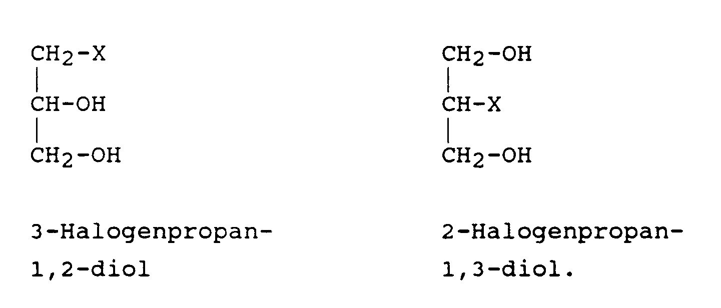
Die Halogenpropandiole sind ebenfalls Flüssigkeiten. Da Chlor das übliche Halogen ist, stellt das 3-Chlorpropan-1,2-diol, dessen Siedepunkt unter Normaldruck bei 213 °C liegt, die bevorzugte Verbindung dar. Bei dem nach dem erfindungsgemäßen Verfahren hergestellten Dialkylaminopropandiol handelt es sich demnach vorzugsweise um ein Dimethylaminopropandiol, und zwar um das 3-Dimethylaminopropan-1,2-diol.

Das erfindungsgemäße Verfahren weist gegenüber dem Stand der Technik eine Reihe von Vorteilen auf. Die Umsetzung kann mit einer wesentlich höheren Konzentration an Dialkylamin oder anders ausgedrückt mit einem wesentlich geringeren Wasseranteil durchgeführt werden. Ferner ist die Reaktionszeit viel kürzer. Aufgrund der höheren Dialkylamin-Konzentration und der kürzeren Reaktionszeit wird eine hohe Raum-Zeit-Ausbeute erreicht. Auch die chemische Ausbeute an Dialkylaminopropandiol, bezogen auf eingesetztes Glycerin-monohalogenhydrin, ist sehr groß. Sie liegt in der Regel bei 91 bis 97 %. Ein weiterer Vorteil des erfindungsgemäßen Verfahrens ist die hohe Reinheit des erhaltenen Dialkylaminopropandiols. Es kann vielfach ohne destillative Aufarbeitung weiterverwendet werden. Bei einer destillativen Feinreinigung fällt nur sehr wenig Rückstand an. Die genannten Vorteile ergeben sich auch dann, wenn von einem rohen Halogenpropandiol ausgegangen wird, das ist zum Beispiel ein Chlorpropandiol, wie es durch Hydrolyse von Epichlorhydrin erhalten wird, und zwar ohne weitere Reinigung des Hydrolyseproduktes, dieses kann vielmehr als solches eingesetzt werden. All diese überraschenden Effekte des erfindungsgemäßen Verfahrens resultieren offenbar aus der Kombination der beschriebenen Maßnahmen. Das erfindungsgemäße Verfahren ist ferner diskontinuierlich oder kontinuierlich durchführbar.

Dialkylaminopropandiole, wie Dimethyl- und Diethylaminopropandiol, stellen aufgrund ihrer Trifunktionalität wertvolle Zwischenprodukte dar.

Die Erfindung wird nun an Beispielen noch näher erläutert.

### Beispiel 1

900,0 g (20,0 mol) Dimethylamin und 385,7 g Wasser (das sind 30,0 Gew.-% Wasser, bezogen auf Dimethylamin plus Wasser) wurden in einem Reaktionsgefäß vorgelegt und unter Rühren auf 30 °C erwärmt (auch die folgenden Operationen erfolgten unter Rühren; das gilt auch für alle weiteren Beispiele). Zu dieser Mischung wurden 734,8 g (6,65 mol) 3-Chlorpropan-1,2-diol im Verlauf von etwa 2 Stunden unter Aufrechterhalten einer Temperatur von 30 bis 35 °C kontinuierlich dazugegeben. Nach der Zugabe des Chlorpropandiols wurde die Mischung bei der genannten Temperatur noch weitere 7 Stunden zur Nachreaktion gehalten, worauf alles Chlorpropandiol umgesetzt war (der Druck im Reaktionsgefäß, der im wesentlichen vom Dampfdruck des Dimethylamins resultiert, lag bei 0,15 bis 0,20 MPa).

Die Aufarbeitung des Reaktionsproduktes wurde wie folgt durchgeführt: Vom Reaktionsprodukt (Inhalt des Reaktionsgefäßes) wurde zunächst das überschüssige Dimethylamin und der Großteil des Wassers bei Normaldruck und einer Temperatur von höchstens 115 °C abdestilliert.
Das weitere Abdestillieren des Wassers erfolgte im Wasserstrahlvakuum bei 0,005 MPa. Die nun vorliegende Mischung aus Dimethylaminopropanhydrochlorid und Wasser (etwa 2 Gew.-%) wurde zur Freisetzung des Dimethylaminopropandiols vom Hydrochlorid mit einer stöchiometrischen Menge Natriumhydroxid, bezogen auf eingesetztes Chlorpropandiol (das sind 6,65 mol NaOH), in Form einer etwa 50gew.%igen Lösung unter Rühren und Aufrechterhalten einer Temperatur von höchsten 50 °C innerhalb von 1 Stunde versetzt. Von der aus Dimethylaminopropandiol, Wasser und ausgefallenem Natriumchlorid bestehenden Mischung wurde das ausgefallene Salz durch Filtration bei Raumtemperatur abgetrennt. Der Filterkuchen wurde dreimal mit Isopropanol gewaschen (250 ml, 200 ml und 150 ml), worauf die einzelnen Filtrate vereinigt wurden. Von der Mischung aus Dimethylaminopropandiol, Wasser und Isopropanol wurde das Wasser und das Isopropanol unter Wasserstrahlvakuum bei einer Temperatur von bis zu 100 °C abdestilliert.
Anschließend wurde das 3-Dimethylaminopropan-1,2-diol vom Siedepunkt 74 bis 75 °C bei 122 Pa überdestilliert. Es wurden 734,3 g (6,16 mol) reines Produkt erhalten, das sind 92,6 % der theoretischen Ausbeute. Das Produkt hatte eine Reinheit von 99,8 %.

### Beispiel 2

900,0 g (20,0 mol) Dimethylamin und 385,7 g Wasser (das sind 30,0 Gew.-% Wasser, bezogen auf Dimethylamin plus Wasser) wurden in einem Reaktionsgefäß vorgelegt und unter Rühren auf 40 °C erhitzt. Zu dieser Mischung wurden 553,0 g (5,0 mol) 3-Chlorpropan-1,2-diol im Verlauf von etwa 1,5 Stunden unter Aufrechterhalten einer Temperatur von 45 bis 50 °C kontinuierlich dazugegeben. Nach der Zugabe des Chlorpropandiols wurde die Mischung bei der genannten Temperatur noch weitere 5 Stunden zur Nachreaktion gehalten, worauf alles Chlorpropandiol umgesetzt war (der Druck im Reaktionsgefäß lag bei circa 0,2 MPa).
Die Aufarbeitung des Reaktionsproduktes erfolgte analog Beispiel 1. Es wurden 559,0 g (4,69 mol) 3-Dimethylaminopropan-1,2-diol vom Siedepunkt 93 °C bei 0,7 bis 0,9 kPa erhalten, das sind 93,8 % der Theorie. Das Dimethylaminopropandiol hatte eine Reinheit von 99,5 %.

### Beispiel 3

900,0 g (20,0 mol) Dimethylamin, 900,0 g Wasser (das sind 50,0 Gew.-% Wasser, bezogen auf Dimethylamin plus Wasser) und 443,0 g (4,0 mol) 3-Chlorpropan-1,2-diol wurden in einem Reaktionsgefäß unter Rühren und Kühlen vorgelegt und auf einer Temperatur von 50 bis 55 °C eingestellt. Die Mischung wurde bei der genannten Temperatur 6 Stunden lang unter Rühren gehalten. Nach dieser Zeit war alles Chlorpropandiol umgesetzt.
Die Aufarbeitung des Reaktionsproduktes erfolgte analog Beispiel 1. Es wurden 464,8 g (3,90 mol) 3-Dimethylaminopropan-1,2-diol vom Siedepunkt 89 °C bei 0,6 bis 0,8 kPa erhalten, das sind 97,5 % der Theorie. Das Dimethylaminopropandiol hatte eine Reinheit von 99,5 %.

### Beispiel 4

443,0 g (4,0 mol) 3-Chlorpropan-1,2-diol wurden in einem Reaktionsgefäß vorgelegt und unter Rühren auf 40 °C erhitzt. Zum erhitzen Chlorpropandiol wurden 900,0 g (20,0 mol) Dimethylamin und 1 350,0 g Wasser (das sind 60 Gew.-% Wasser, bezogen auf Dimethylamin plus Wasser) gleichzeitig und getrennt voneinander im Verlauf von 1 Stunde unter Aufrechterhalten einer Temperatur von 40 bis 45 °C kontinuierlich dazugegeben. Nach dieser Zugabe wurde die Mischung bei der genannten Temperatur noch weitere 4 Stunden zur Nachreaktion gehalten, worauf alles Chlorpropandiol umgesetzt war.
Die Aufarbeitung des Reaktionsproduktes erfolgte analog Beispiel 1. Es wurden 463,6 g (3,89 mol) 3-Dimethylaminopropan-1,2-diol vom Siedepunkt 93 °C bei 0,7 bis 0,9 kPa erhalten, das sind 97,3 % der Theorie. Das Dimethylaminopropandiol hatte eine Reinheit von 99,5 %.

### Beispiel 5

443,0 g (4,0 mol) 3-Chlorpropan-1,2-diol wurden in einem Reaktionsgefäß vorgelegt und unter Rühren auf 35 °C erhitzt. Zum erhitzen Chlorpropandiol wurden 450,0 g (10,0 mol) Dimethylamin und 675,0 g Wasser (das sind 60 Gew.-% Wasser, bezogen auf Dimethylamin plus Wasser) gleichzeitig und getrennt voneinander im Verlauf von 1,5 Stunden unter Aufrechterhalten einer Temperatur von 35 bis 40 °C kontinuierlich dazugegeben. Nach dieser Zugabe wurde die Mischung bei der genannten Temperatur noch weitere 5 Stunden zur Nachreaktion gehalten, worauf alles Chlorpropandiol umgesetzt war.
Die Aufarbeitung des Reaktionsproduktes erfolgte analog Beispiel 1. Es wurden 435,1 g (3,65 mol) 3-Dimethylaminopropan-1,2-diol vom Siedepunkt 89 °C bei 0,6 bis 0,8 kPa erhalten, das sind 91,3 % der Theorie. Das Dimethylaminopropandiol hatte eine Reinheit von 99,5 %.

### Vergleichsbeispiel (Nacharbeitung des Beispiels der US-Patentschrift 2,147,226)

3,986 kg von einer 24,5%igen wäßrigen Dimethylamin-Lösung (das sind 0,977 kg oder 21,71 mol Dimethylamin) und 0,679 kg von einer 40%igen wäßrigen Natriumhydroxid-Lösung (das sind 0,272 kg oder 6,80 mol NaOH) wurden in einem Reaktionsgefäß unter Rühren und Kühlen zusammengemischt.
Insgesamt 1,812 kg von Glycerin-1-monochlorhydrin (das sind 16,40 mol) wurden in 0,091-kg-Portionen in jeweils 15 Minuten hinzugefügt. Die Reaktionsmischung wurde die ganze Zeit hindurch bei 20 bis 40 °C gehalten. Der Druck im Reaktionsgefäß stieg bis auf 0,1 MPa. Die Mischung wurde 24 Stunden lang stehengelassen, worauf 0,679 kg von einer 40%igen wäßrigen Natriumhydroxid-Lösung (das sind 0,272 kg oder 6,80 mol NaOH) dazugegeben wurden. Das Reaktionsgefäß wurde nun geöffnet und die Mischung stufenweise auf 105 °C erhitzt, wobei das nicht reagierte Dimethylamin abdestillierte. Das Wasser wurde aus der verbliebenen Mischung bei einem Druck von 6,7 kPa abdestilliert. Der Destillationsrückstand wurde auf 40 °C abgekühlt und mit 1,520 kg Methanol gemischt, wobei Natriumchlorid aus der Mischung ausfiel. Das Salz wurde in einer Zentrifuge abgetrennt, worauf das Methanol von der Flüssigkeit durch Destillation bei einem Druck von 6,7 kPa zurückgewonnen wurde. Es wurden 1,295 kg (10,86 mol) 3-Dimethylaminopropan-1,2-diol vom Siedepunkt 74 bis 75 °C bei 122 Pa erhalten, das sind 66,3 % der Theorie.

## Patentansprüche

1. Verfahren zur Herstellung von Dialkylaminopropandiol durch Umsetzung von Monohalogenpropandiol mit überschüssigem Dialkylamin in Gegenwart von Wasser, dadurch gekennzeichnet, daß das Dialkylamin in einer Menge von mindestens 2,5 mol pro mol Monohalogenpropandiol eingesetzt und die Umsetzung in Gegenwart von 25 bis 70 Gew.-% Wasser, bezogen auf die Mischung aus Dialkylamin und Wasser, und bei einer Temperatur von 20 bis 80 °C durchgeführt wird und das Dialkylaminopropandiol aus dem Umsetzungsprodukt gewonnen wird.

2. Verfahren nach Anspruch 1, dadurch gekennzeichnet, daß das Dialkylamin in einer Menge von 3 bis 6 mol pro mol Monohalogenpropandiol eingesetzt wird und die Umsetzung in Gegenwart von 30 bis 60 Gew.-% Wasser und bei einer Temperatur von 30 bis 60 °C durchgeführt wird.

3. Verfahren nach Anspruch 1 oder 2, dadurch gekennzeichnet, daß als Dialkylamin Dimethylamin oder Diethylamin und als Monohalogenpropandiol Monochlorpropandiol eingesetzt wird.

## Claims

1. A process for the preparation of dialkylaminopropanediol by reaction of monohalopropanediol with excess dialkylamine in the presence of water, which comprises employing the dialkylamine in an amount of at least 2.5 mol per mole of monohalopropanediol and carrying out the reaction in the presence of 25 to 70% by weight of water, relative to the mixture of dialkylamine and water, and at a temperature of 20 to 80°C and recovering the dialkylaminopropanediol from the reaction product.

2. The process as claimed in claim 1, wherein the dialkylamine is employed in an amount of 3 to 6 mol per mole of monohalopropanediol and the reaction is carried out in the presence of 30 to 60% by weight of water and at a temperature of 30 to 60°C.

3. The process as claimed in claim 1 or 2, wherein dimethylamine or diethylamine is employed as the dialkylamine and monochloropropanediol is employed as the monohalopropanediol.

## Revendications

1. Procédé de préparation de dialkyl-aminopropanediols par réaction d'un monohalogénopropane-diol avec un excès d'une dialkylamine en présence d'eau, procédé caractérisé en ce que l'on opère avec une proportion de la dialkylamine d'au moins 2,5 moles par mole du monohalogénopropane-diol et en ce que l'on effectue la réaction en présence de 25 à 70% en poids d'eau, par rapport au mélange de la dialkylamine et de l'eau, et à une température de 20 à 80°C, puis on récupère le dialkylaminopropane-diol du produit de la réaction.

2. Procédé selon la revendication 1, caractérisé en ce que l'on opère avec une proportion de la dialkylamine de 3 à 6 moles par mole du monohalogénopropane-diol et en ce que l'on effectue la réaction en présence de 30 à 60% en poids d'eau et à une température de 30 à 60°C.

3. Procédé selon la revendication 1 ou 2, caractérisé en ce que la dialkylamine est la diméthylamine ou la diéthylamine et le monohalogénopropane-diol est le monochloropropane-diol.
